# EUROPÄISCHE PATENTSCHRIFT

(11) **EP 1 997 380 B1**
(45) Veröffentlichungstag und Bekanntmachung des Hinweises auf die Patenterteilung: **16.11.2016**
(21) Anmeldenummer: 08154335.7
(22) Anmeldetag: 10.04.2008
(51) Int. Cl.: A01N 43/16, A01N 65/00, A01N 37/38, A01N 49/00, A61Q 17/02, A61K 8/97, A01P 17/00

(54) **Insektenschutzmittel und Parfum**
Insect repellent and perfume
Insectifuge et parfum

(30) Priorität: 31.05.2007 DE 102007026048
(43) Veröffentlichungstag der Anmeldung: 03.12.2008
(73) Patentinhaber: Beiersdorf Aktiengesellschaft, 20245 Hamburg (DE)
(72) Erfinder: Treu, Jens, Dr., 22844 Norderstedt (DE); Hamer, Gunhild, 22455 Hamburg (DE); Zilz, Werner, 25469 Halstenbek (DE); von der Fecht, Stephanie, 22880 Wedel (DE); Nielsen, Jens, 24558 Henstedt-Ulzburg (DE); Schulz, Jens, Dr., 25462 Rellingen (DE); Kröpke, Rainer, 22869 Schenefeld (DE)
(74) Vertreter: Hartmann, Jost

(56) Entgegenhaltungen:
- EP-A- 1 745 772
- WO-A-03/062357
- WO-A-2006/050519
- WO-A-2007/133683
- US-A1- 2004 197 364
- HARKENTHAL M ET AL: "COMPARATIVE STUDY ON THE IN VITRO ANTIBACTERIAL ACTIVITY OF AUSTRALIAN TEA TREE OIL, CAJUPUT OIL, NIAOULI OIL, MANUKA OIL, KANUKA OIL, AND EUCALYPTUS OIL", DIE PHARMAZIE, GOVI VERLAG PHARMAZEUTISCHER VERLAG GMBH, ESCHBORN, DE, vol. 54, no. 6, 1 June 1999 (1999-06-01), pages 460-463, XP001061666, ISSN: 0031-7144
- CLARK AND R C MENARY R J: "Variations in Composition of Peppermint Oil in Relation to Production Areas", ECONOMIC BOTANY, NEW YORK BOTANICAL GARDEN, BRONX, NY, US, vol. 35, no. 1, 1 January 1981 (1981-01-01), pages 59-69, XP009161156, ISSN: 0013-0001
- CHRIS PETERSON ET AL: 'Insect repellents - past, present and future' PESTICIDE OUTLOOK Bd. 12, Nr. 4, 01 August 2001, Seiten 154 - 158, XP055127984 DOI: 10.1039/b106296b ISSN: 0956-1250
- U. BERNIER ET AL: 'Comparison of Contact and Spatial Repellency of Catnip Oil and N,N-Diethyl-3-methylbenzamide (Deet) Against Mosquitoes' JOURNAL OF MEDICAL ENTOMOLOGY Bd. 42, Nr. 3, 01 Mai 2005, Seiten 306 - 311, XP055127985

## Beschreibung

Die vorliegende Erfindung betrifft kosmetische Formulierungen zur Insektenabwehr umfassend einen oder mehrere Insektenrepellentwirkstoffe gewählt aus Dihydronepetalacton und/oder Extrakten der Katzenminze und ein oder mehrere Parfuminhaltststoffe.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Als Repellent-Wirkstoff ist beispielsweise 1-Piperidincarboxylsäure 2-(2-hydroxyethyl)-1-methylpropylester (INN: Icaridin, CAS-Nummer: 119515-38-7, Elincs-Nummer: 423-210-8) bekannt, der die folgende Struktur aufweist:

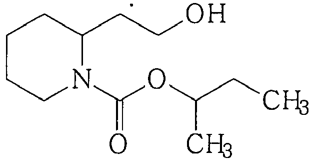

Ein weiterer eingesetzter Repellent-Wirkstoff ist der 3-(N-n-Butyl-N-acetyl-amino)propion-säureethylester (auch als Ethylbutylacetylaminopropionat oder Repellent 3535 bezeichnet), welcher sich durch die folgende Strukturformel auszeichnet:

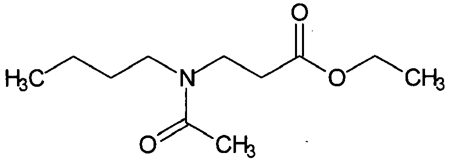

Nicht zuletzt kennt der Fachmann den Repellent-Wirkstoff N,N-Diethyl-3-methylbenzamid (Handelsbezeichnung: Meta-delphene, DEET), der die folgende Struktur aufweist:

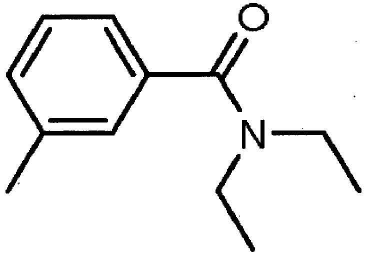

Diese Stoffe sind als Repellentwirkstoffe gegen Insekten, insbesondere Mücken oder Zecken, vielfach beschrieben, wie z.B. in DE 2246433, DE 19645250, DE 19645920.

In der WO 2006096876 und WO 2005034626 werden Dihydronepetalactone als Repellentien gegen Insekten offenbart.

Katzenminze oder auch Nepeta ist als Mückenabwehrmittel bekannt und soll eine etwa zehn Mal stärkere Abwehr gegen Mücken als herkömmliche Abwehrmittel aufweisen.

WO 03062357 beschreibt eine Parfumzusammensetzung, die aus Dihydronepetalacton besteht und angenehme Dufteigenschaften aufweisen soll. Mit dieser Parfumzusammensetzung werden weiterhin eine Hautbehandlung sowie eine Methode zur Auftragung auf die Haut beschrieben, eine Verwendung dieser Zusammensetzung zur Insektenabwehrwird nicht erwähnt.

EP 1745772 beschreibt eine kosmetische Zubereitung, die aus einem Insektenabwehrmittel ausgewählt aus der Gruppe 2-(Z-Hydroxyethyl)-1-methylpropylester (Icardin), 3-(N-n-Butyl-N-acetylamino)propionsäureethylester oder N,N-Diethyl-3-methylbenzamid (DEET), und einer oder mehreren Verbindungen ausgewählt aus der Gruppe umfassend insgesamt 16 Verbindungen, wobei Hexylsalicylat, Linalylacetat, 2-lsobutyl-4-hydroxy-4-methyltetrahydropyran genannt sind, besteht. WO 2006050519 beschreibt eine insektenabwehrende Zusammensetzung, die aus Dihydronepetalacton, einem Alkohol und einem Ester besteht.

US 20040197364 beschreibt eine Zusammensetzung zur Insektenabwehr, die aus verschiedenen ätherischen Ölen besteht, darunter Eukalyptusö|, Extrakt der Katzenminze, Aloe Vera, Jojobaö|, Teebaumöl und Pfefferminzöl.

In der Literatur (Harkenthal M. et al; Die Pharmazie, Bd. 54, Nr.6 1. Juni 1999, Seiten 460-463; Clark et al., Economic Botany, Bd. 35, Nr.1 1. Januar 1981, Seiten 59-69;Chris Peterson et al., Pesticide Outlook, Bd. 12, Nr. 4, August 2001, Seiten 154 - 158 und U. Bernier et al., Journal of Medical Entomology, Bd. 42, Nr. 3, 1. Mai 2005, Seiten 306-311) werden verschiedenste Insektenabwehrmittel beschrieben. U.a. wird ausgeführt, dass Extrakte der Katzenminze eine stärkere Abwehrwirkung als DEET aufweisen.

In der DE 102005033845 werden des weiteren u.a. bestimmte Parfuminhaltsstoffe zur Abwehr von Mücken, Sandfliegen, Läusen, Bremsen, Wespen, Bienen, Fliegen und Zecken erwähnt.

Ein Problem ist dabei, dass ein Repellentwirkstoff, der beispielweise gegen Mücken abwehrend wirkt, diese Abwehrwirkung nicht oder nur eingeschränkt gegen andere Insekten aufweist.

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil an Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repellent-Wirkstoffen einzusetzen.

Die Repellent-Wirkstoffe haben zudem einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Die Zubereitungen des Standes der Technik haben bei insbesondere hohen Einsatzkonzentrationen den Nachteil, insbesondere bei Menschen mit empfindlicher oder zu Allergien neigender Haut nicht besonders verträglich zu sein und in Einzelfällen zu Hautreizungen zu führen.

Wünschenswert ist es eine Zubereitung zur Verfügung zu stellen, die einerseits insektenabwehrend wirkt und andererseits hautverträglich gestaltet ist und einen für die menschliche Nase angenehm wohlriechenden Duft verströmt.

Es war daher die Aufgabe der vorliegenden Erfindung, kosmetische Zubereitungen mit einem Gehalt an Dihydronepetalactone zu entwickeln, welche mit einer reduzierten Menge an Repellent-Wirkstoffen zu einer gleichen Wirkung wie herkömmliche Insektenabwehrmittel kommen und die ein reduziertes Hautreizungspotential aufweisen. Nicht zuletzt war des die Aufgabe ein Insektenabwehrmittel zu entwickeln, welches einen für die menschliche Nase angenehm wohlriechenden Duft verströmt.

Die Erfindung beschreibt eine Zubereitung umfassend einen oder mehrere Insektenrepellentwirkstoffe gewählt aus Dihydronepetalactonen und/oder Extrakten der Katzenminze und ein oder mehrere Parfuminhaltsstoffe gewählt aus der Gruppe Hexylsalicylat und Linalylacetat.

Vorteilhaft ist der Zusatz von Hexylsalicylat, Linalylacetat und 2-Isobutyl-4-hydroxy-4-methyltetrahydropyran, die den Eigengeruch des Dihydronepetalactons besonders gut maskieren ohne einen anlockenden Effekt auf die Insekten auszuüben.

Die erfindungsgemäßen Parfuminhaltstoffe, ein oder mehrere, sind bevorzugt zu einer Gesamtkonzentration von 1 ppm bis 2,5 Gew.%, jeweils bezogen auf das Gesamtgewicht der Zubereitung, in dieser enthalten.

Eine vorteilhafte Ausführungsform im Sinne der vorliegenden Erfindung ist eine kosmetische Zubereitung als Hydrodispersionsgel enthaltend 15 Gew.% Dihydronepetalacton und 0,004 Gew.% Hexylsalicylat, jeweils bezogen auf das Gesamtgewicht der Zubereitung. Insbesondere ist der Anteil an Repellentien in der Zubereitung im Bereich von 5 Gew% bis 40 Gew%, bezogen auf die Gesamtmasse der Zubereitung, zu wählen. Dihydronepetalactone sind beispielweise in der WO 2006096876 und WO 2005034626 beschrieben.

Die Katzenminze (Nepeta) bezeichnen eine Pflanzengattung der Familie der Lippenblütler (Lamiaceae). Erfindungsgemäß werden als Extrakte alle möglichen Extrakte, Lösungen oder Dispersionen der Nepetapflanze oder deren Teile verstanden.

Bei den in den Nepeta enthaltenen ätherischen Ölen handelt es sich in den Hauptbestandteilen um Citral, Citronellol, Geraniol und Limonen, sowie Nepetalacton, Gerb- und Bitterstoffe. Der Wirkstoff, der die Pflanze für Katzen so unwiderstehlich macht, ist Actinidin, ein iridoides Glykosid, das dem vergleichbaren Wirkstoff des Baldrian sehr ähnlich ist (BOWN, 1995). Die Nepeta enthält etwa 0,2-0,7 % ätherische Öle.

Der ölige Extrakt der Katzenminze, insbesondere aufgereinigt, ist wie jedes andere Naturöl sehr schlecht spreitend und sehr polar. Es ist deshalb unkosmetisch und verleiht den herkömmlichen kosmetischen Formeln eine ölig, fettige Phase. Es wird als schwer verteilbar wahrgenommen. Der polare Charakter des Extraktes führt zudem sehr schnell zu Instabilitäten, wie z.B. Öl- oder Wasserabscheidung.

Die erfindungsgemäße Zubereitung kann die Dihydronepetalactone und/oder Extrakte der Katzenminze in unterschiedlichen Formen enthalten. Bevorzugt enthält die Zubereitung eine aufgereinigte Repellentienmischung, die zu etwa 90 Gew.% Dihydronepetalacton und 2 Gew.% Dehydronepetalacton, bezogen auf die Mischungsmasse, als wirksame Repellentien umfasst.

Es ist erfindungsgemäß vorteilhaft, wenn die Repellentien in einer Gesamtkonzentration von 0,1 bis 40 Gew.% und erfindungsgemäß bevorzugt, wenn die Repellentien in einer Gesamtkonzentration von 5 bis 20 Gew.%, jeweils bezogen auf das Gesamtgewicht der Zubereitung, in dieser enthalten sind.

Bevorzugt ist eine erfindungsgemäße Zubereitung, die weiteren Insektenrepellentien, wie z.B. 1-Piperidincarboxylsäure 2-(2-hydroxyethyl)-1-methylpropylester (INN: Icaridin) und/oder N,N-Diethyl-3-methylbenzamid (DEET) und/oder Ethylbutylacetylaminopropionat (EBAAP) umfasst.

Neben der Insektenabwehr sind aber vor allem die Hautverträglichkeit und das Hautgefühl entscheidende Eigenschaften eines topisch zu applizierenden insbesondere kosmetischen Produktes.

Es nützt eine hervorragende Repellentwirksamkeit nichts, wenn das Kosmetikprodukt zu Unverträglichkeiten führt.
Eine der häufig auftretenden Hautunverträglichkeiten ist der sog. Stinging-Effekt, der sich insbesondere im Gesicht unangenehm bemerkbar macht. Der Stinging-Effekt tritt insbesondere im Nasolabial-Bereich auf und bedeutet ein subjektives Missempfinden der Probanden.
Um solche unerwünschten Effekte für Kosmetika möglichst auszuschliessen werden vor Produkteinführung sog. Stinging-Tests durchgeführt. Jeweils zwei Produkte werden dabei im dirketen Paarvergleich auf ihre Hautverträglichkeit getestet. Als interner High-Standard (Benchmark) wird dabei ein als hautverträglich bekanntes handelsübliches Produkt verglichen (NIVEA Visage Day Cream normal mixed).

In zahlreichen Untersuchungen wurde nun festgestellt, dass ein wesentlicher Beitrag zum Stinging durch die in vielen Kosmetika enthaltenen Parabene hervorgerufen wird, insbesondere wenn diese mit einem Anteil von mehr als 0,3 Gew.% in der Rezeptur vorliegen.
Parabene werden als Konservierungsmittel eingesetzt, so dass deren Ersatz mitunter schwierig ist.

Es wurde nun überaschenderweise fest gestellt, dass die erfindungsgemäßen Zubereitungen sehr gute Repellentwirksamkeiten zeigen aber einen Stinging-Effekt nicht aufweisen.
Die erfindungsgemäßen Zubereitungen lassen sich daher bevorzugt mit einem Anteil von bis zu 0,3 Gew.% Konservierungsmittel, wie insbesondere Parabene, herstellen.
Bevorzugt kann auf den Zusatz von Konservierungsmitteln, wie z.B. Parabenen, ganz verzichtet werden. D.h es ist erstmals möglich Repellenthaltige Zubereitungen zur Verfügung zu stellen, die gleichzeitig verbesserte Verträglichkeit aufweisen und insbesondere keine Stinging-Effekte aufweisen.
Die erfindungsgemäßen Zubereitungen sind daher bevorzugt frei von bakteriziden-, fungiziden-, sporizid d.h. sporenabtötenden, wirksamen Mitteln und/oder frei von Konservierungsmitteln. Weiterhin sind sie bevorzugt frei von Formaldehyd, Formaldehyddonatoren wie Diazolidinyl urea, Imidazolidinyl urea und/oder DMDM Hydantoin sowie insbesondere frei von Benzoesäure, p-Hydroxybenzoesäure und/oder oder deren Derivate oder Salze. Bevorzugt umfassen die erfindungsgemäßen Zubereitungen weniger als 0,3 Gew.% bzw. insbesondere sind sie frei von Parabenen, insbesondere frei von Methylparaben, Ethylparaben, Propylparaben, Isopropylparaben, Butylparaben, Isobutylparaben und/oder Benzylparaben. Auch 2,4-Dichlorobenzylalkohol, 4-Chloro-3,5-dimethyl-phenol, 2-Bromo-2-nitropropane-1,3-diol und/oder lodopropinylbutylcarbamate (IPBC) sowie Quaternium-15 (CAS 51229-78-8), Methyl-chloroisothiazolinone und/oder Methylisothiazolinon sind in den erfindungsgemäßen Zubereitungen nicht bzw. zu weniger als 0,3 Gew.% zu finden.
Die erfindungsgemäßen Zubereitungen sind besonders bevorzugt Parabenfrei.
Es bedeutet erfindungsgemäß "frei von" ein Anteil von weniger als 0,1 Gew.%, bezogen auf die Gesamtmasse der Zubereitung. Bevorzugt beträgt der Anteil an den zuvor genannten Mitteln und insbesondere Parabenen 0 Gew.%.

Die Zubereitung ist bevorzugt eine kosmetische Zubereitung und zum Auftragen auf die Haut geeignet.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/S, S/W oder multiple Emulsion, Makro- oder Mikroemulsion), einer Dispersion, einer Pickering-Emulsion, eines Gels, eines Hydrodispersionsgels oder einer wasserfreien Zubereitung vorliegt. Dabei ist es erfindungsgemäß bevorzugt, wenn die kosmetische Zubereitung in Form eines Hydrodispersionsgels vorliegt.

Die Zubereitung kann erfindungsgemäß auch in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Zubereitungen mit Dihydronepetalacton als Wirkstoff können in Form von Lösungen, Emulsionen und Dispersionen hergestellt werden, bei denen der lipophile Wirkstoff von einer wässrigen Phase umgeben ist (z.B. O/W-Emulsion). Durch die Umhüllung des Wirkstoffs mit der wässrigen Phase wird dessen Freisetzung aber gebremst und die Zubereitung selbst ist bei der Anwendung auf der Haut länger wirksam gegen Mücken, Insekten, etc.

Nachteilig am Stande der Technik ist jedoch der Sachverhalt, das diese Zubereitungen aufgrund ihrer wässrigen äußeren Phase nicht besonders wasserfest sind und leicht von der Haut abgespült werden können. Ferner lassen sich in diesen Zubereitungen keine oxidationsempfindlichen, wasserlöslichen Zusatzstoffe einarbeiten.

Diese Mängel lassen sich erfindungsgemäß durch Emulsionen mit einer wässrigen inneren Phase und einer lipophilen äußeren Phase, enthaltend Dihydronepetalactone, beheben.

Besonders vorteilhaft liegen die erfindungsgemäßen Zubereitung daher in Form einer W/O-oder W/S-Emulsion vor.

Die erfindungsgemäßen Zubereitungen weisen dadurch eine verlängerte Wirkdauer und eine erhöhte Wasserfestigkeit auf der Haut auf.

Überraschend war insbesondere der Umstand, dass, obwohl der Repellent-Wirkstoff in der äußeren, lipophilen Phase angereichert ist, bei der Anwendung auf der Haut gleichmäßig über einen Zeitraum von mehreren Stunden an die Umwelt abgegeben wird und somit eine lang anhaltende Repellent-Wirkung entfaltet.

Überraschend war nicht, zuletzt, dass bei den erfindungsgemäßen Zubereitungen die Klebrigkeit der Zubereitung auf der Haut im Vergleich zu Produkten des Stand der Technik (mit gleichem Repellent-Gehalt) deutlich reduziert ist.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfingdungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Als insbesondere kosmetische Zubereitung ist die erfindungsgemäße Zubereitung als topisch anzuwendendes Mittel vorteilhaft geeignet.

Ebenso ist die Verwendung einer erfindungsgemäßen Zubereitung als Insektenabwehrmittel in einer Verdunstungsvorrichtung möglich und bevorzugt.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**W/O-Emulsionen**

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Cellulose | 0,5 | 1,0 | --- | 0,25 | 2,5 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Hexylsalicylat | 0,125 | 0,005 | 0,04 | 1,0 | 0,75 |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Dihydronepetalacton | 5 | 7,5 | 10 | 12,5 | 30 |
| Wasser | ad 100 | ad 100 | ad 100 | ad 100 | ad 100 |

### W/O-Emulsionen

| | **6** | **7** | **8** | **9** | **10** |
|---|---|---|---|---|---|
| PEG-30 Dipolyhydroxystearat | --- | 0,5 | 0,25 | --- | 8,0 |
| Lanolin Alcohol | 1,0 | 1,5 | 1,75 | 3,0 | --- |
| Paraffinöl | 12,5 | 10,0 | 8,0 | 5,0 | 12,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Linaylcetat | 0,15 | 15 ppm | 0,5 | 0,0025 | 0,0065 |
| Dihydronepetalacton + Katzenminzeextrakt | 7,5 | 12,5 | 17,5 | 20 | 35 |
| Wasser | ad 100 | ad 100 | ad 100 | ad 100 | ad 100 |

### Silikon in Wasser-Emulsion

| | **31** | **32** | **33** | **34** | **35** |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Linalylacetat | 0,1 | 40 ppm | 0,08 | 0,0045 | 0,15 |
| Dihydronepetalacton | 10 | 20 | 30 | 40 | 15 |
| Xanthan Gum | --- | 0,1 | --- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Methylisothiazolinone | --- | --- | 0,05 | --- | 0,1 |
| Wasser | ad 100 | ad 100 | ad 100 | ad 100 | ad 100 |

### O/W-Emulsion

| | **41** | **42** | **43** | **44** | **45** |
|---|---|---|---|---|---|
| Polyethylenglycol(21)stearylether | 1 | -- | 2,5 | 2 | 1,5 |
| Polyethylenqlycol(2)stearylether | 1 | -- | 5,5 | 3 | 7,5 |
| Cetearylqlucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Dihydronepetalacton + Katzenminzeextrakt | 10, | 20 | 15 | 7,5 | 40 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Methylisothiazolinone | --- | --- | 0,05 | --- | 0,1 |
| Hexylsalicylat | 0,1 | 20 | 0,0065 | 0,0015 | 0,5 |
| | | ppm | | | |
| Wasser | ad 100 | ad 100 | ad 100 | ad 100 | ad 100 |

### O/W-Emulsion

| | **46** | **47** | **48** | **49** | **50** |
|---|---|---|---|---|---|
| Glycerylsteratcitrat | 1,0 | 0,5 | 1,5 | 9,0 | 0,3 |
| Polyethylenglycol(20)cetearylether | 10,0 | 1,0 | 5 | --- | --- |
| Triglycerinmethylglucosedistearat | --- | --- | 3,5 | --- | 2,5 |
| Linalylacetat | 0,75 | 1,5 | 2,25 | 0,125 | 0,035 |
| Katzenminzeextrakt | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Methylisothiazolinone | --- | --- | 0,05 | --- | 0,1 |
| Phenoxyethanol | --- | 0,5 | --- | 0,15 | --- |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

### Pumpspray

| | **56** | **57** | **58** | **59** | **60** |
|---|---|---|---|---|---|
| Polyethylenglycol(40)stearat | 1,0 | --- | 0,5 | --- | --- |
| Cyclomethicon | 0,5 | --- | --- | --- | --- |
| Dihydronepetalacton | 2 | 10 | 15 | 20 | 40 |
| Parfum | q,s, | q,s, | q,s, | q,s, | q,s, |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extrakt | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Linalylacetat | 0,015 | 0,0065 | 35ppm | 0,15 | 0,0095 |
| Wasser | ad 100 | ad 100 | ad 100 | ad 100 | ad 100 |

### Spray

| | **61** | **62** | **63** | **64** | **65** |
|---|---|---|---|---|---|
| Ethylbutylacetylaminopropionat | 5 | 35 | 15 | 20 | 7,5 |
| N, N-Diethyltoluolamid | 5 | --- | --- | 1 | --- |
| Dihydronepetalacton | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extrakt | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Hexylsalicylat | 0,1 | 0,015 | 0,5 | 75 ppm | 0,0065 |
| Parfum | q,s, | q,s, | q,s, | q,s, | q,s, |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Zubereitung umfassend einen oder mehrere Insektenrepellentwirkstoffe gewählt aus Dihydronepetalactonen und/oder Extrakten der Katzenminze und ein oder mehrere Parfuminhaltsstoffe gewählt aus der Gruppe Hexylsalicylat und Linalylacetat.

2. Zubereitung nach Anspruch 1 **dadurch gekennzeichnet, dass** die Zubereitung mehrere Parfuminhaltsstoffe gewählt aus der Gruppe Hexylsalicylat, Linalylacetat und 2-Isobutyl-4-hydroxy-4-methyltetrahydropyran umfasst.

3. Zubereitung nach Anspruch 1 oder 2 **dadurch gekennzeichnet, dass** der Anteil an Repellentien 5 Gew% und mehr umfasst, bezogen auf die Gesamtmasse der Zubereitung.

4. Zubereitung nach einem der vorstehenden Ansprüche, **dadurch gekennzeichnet, dass** die Zubereitung eine aufgereinigte Repellentienmischung, die zu 90 Gew.% Dihydronepetalacton und 2 Gew.% Dehydronepetalacton umfasst, bezogen auf die Mischungsmasse, enthält.

5. Zubereitung nach einem der vorstehenden Ansprüche, **dadurch gekennzeichnet, dass** die Zubereitung ein oder mehrere weitere Insektenrepellentien enthält.

6. Zubereitung nach einem der vorstehenden Ansprüche, **dadurch gekennzeichnet, dass** die Zubereitung maximal 0,3 Gew.% bakterizide-, fungizide-, sporizid wirksame Mittel, Konservierungsmittel und/oder Formaldehyd und Formaldehyddonatoren enthält.

7. Zubereitung nach einem der vorstehenden Ansprüche, **dadurch gekennzeichnet, dass** die Zubereitung frei von bakteriziden-, fungiziden-, sporizid wirksamen Mitteln, Konservierungsmitteln und/oder Formaldehyd und Formaldehyddonatoren ist.

8. Zubereitung nach einem der vorstehenden Ansprüche, **dadurch gekennzeichnet, dass** die Zubereitung frei von Parabenen ist.

9. Zubereitung nach einem der vorstehenden Ansprüche in Form eines Hydrodispersionsgel.

10. Zubereitung nach einem der vorstehenden Ansprüche 1 bis 8 in Form einer W/O- oder W/S-Emulsion.

11. Zubereitung nach einem der vorstehenden Ansprüche 1 bis 8 in Form einer O/W- oder S/W-Emulsion.

12. Verwendung einer Zubereitung nach einem der Ansprüche 1 bis 11 als Insektenabwehrmittel.

13. Verwendung einer Zubereitung nach einem der Ansprüche 1 bis 11 als Insektenabwehrmittel in einer Verdunstungsvorrichtung.

14. Verwendung einer Zubereitung nach einem der vorstehenden Ansprüche 1 bis 11 als Insektenabwehrmittel **dadurch gekennzeichnet, dass** die Zubereitung als Tränkungsmedium auf Vlies, Pflaster oder Tuch aufgetragen wird.

## Claims

1. Preparation comprising one or more insect repellent active ingredients selected from dihydronepetalactones and/or extracts of cat mint and one or more perfume ingredients selected from the group hexyl salicylate and linalyl acetate.

2. Preparation according to Claim 1, **characterized in that** the preparation comprises a plurality of perfume ingredients selected from the group hexyl salicylate, linalyl acetate and 2-isobutyl-4-hydroxy-4-methyltetrahydropyran.

3. Preparation according to Claim 1 or 2, **characterized in that** the fraction of repellents is 5% by weight and more, based on the total mass of the preparation.

4. Preparation according to one of the preceding claims, **characterized in that** the preparation comprises a purified repellent mixture which comprises 90% by weight dihydronepetalactone and 2% by weight dehydtronepetalactone, based on the mixture mass.

5. Preparation according to one of the preceding claims, **characterized in that** the preparation comprises one or more further insect repellents.

6. Preparation according to one of the preceding claims, **characterized in that** the preparation comprises at most 0.3% by weight of bacteriacidally, fungicidally, sporicidally effective agents, preservatives and/or formaldehyde and formaldehyde donors.

7. Preparation according to one of the preceding claims, **characterized in that** the preparation is free from bacteriacidally, fungicidally, sporicidally active agents, preservatives and/or formaldehyde and formaldehyde donors.

8. Preparation according to one of the preceding claims, **characterized in that** the preparation is free from parabens.

9. Preparation according to one of the preceding claims in the form of a hydrodispersion gel.

10. Preparation according to one of the preceding Claims 1 to 8 in the form of a W/O or W/S emulsion.

11. Preparation according to one of the preceding Claims 1 to 8 in the form of a O/W or S/W emulsion.

12. Use of a preparation according to one of Claims 1 to 11 as insect repellent.

13. Use of a preparation according to one of Claims 1 to 11 as insect repellent in a vaporization device.

14. Use of a preparation according to one of the preceding Claims 1 to 11 as insect repellent, **characterized in that** the preparation is applied as impregnation medium on nonwoven, patch or wipe.

## Revendications

1. Préparation comprenant un ou plusieurs agents actifs insectifuges choisis parmi les dihydronépétalactones et/ou les extraits de cataire et un ou plusieurs ingrédients parfums choisis dans le groupe constitué par le salicylate d'hexyle et l'acétate de linalyle.

2. Préparation selon la revendication 1, **caractérisée en ce que** la préparation comprend plusieurs ingrédients parfum choisis dans le groupe constitué par le salicylate d'hexyle, l'acétate de linalyle et le 2-isobutyl-4-hydroxy-4-méthyltétrahydropyrane.

3. Préparation selon la revendication 1 ou 2, **caractérisée en ce que** la proportion d'agents répulsifs comprend 5 % en poids et plus, par rapport à la masse totale de la préparation.

4. Préparation selon l'une quelconque des revendications précédentes, **caractérisée en ce que** la préparation contient un mélange d'agents répulsifs purifié, qui comprend jusqu'à 90 % en poids de dihydronépétalactone et 2 % en poids de déshydronépétalactone, par rapport à la masse du mélange.

5. Préparation selon l'une quelconque des revendications précédentes, **caractérisée en ce que** la préparation contient un ou plusieurs insectifuges supplémentaires.

6. Préparation selon l'une quelconque des revendications précédentes, **caractérisée en ce que** la préparation contient au plus 0,3 % en poids d'agents à effet bactéricide, fongicide, sporicide, de conservateurs et/ou de formaldéhyde et de donneurs de formaldéhyde.

7. Préparation selon l'une quelconque des revendications précédentes, **caractérisée en ce que** la préparation est exempte d'agents à effet bactéricide, fongicide, sporicide, de conservateurs et/ou de formaldéhyde et de donneurs de formaldéhyde.

8. Préparation selon l'une quelconque des revendications précédentes, **caractérisée en ce que** la préparation est exempte de parabènes.

9. Préparation selon l'une quelconque des revendications précédentes, sous la forme d'un gel d'hydrodispersion.

10. Préparation selon l'une quelconque des revendications 1 à 8 précédentes, sous la forme d'une émulsion E/H ou E/S.

11. Préparation selon l'une quelconque des revendications 1 à 8 précédentes, sous la forme d'une émulsion H/E ou S/E.

12. Utilisation d'une préparation selon l'une quelconque des revendications 1 à 11 en tant qu'agent de lutte contre les insectes.

13. Utilisation d'une préparation selon l'une quelconque des revendications 1 à 11 en tant qu'agent de lutte contre les insectes dans un dispositif de vaporisation.

14. Utilisation d'une préparation selon l'une quelconque des revendications 1 à 11 en tant qu'agent de lutte contre les insectes, **caractérisée en ce que** la préparation est appliquée en tant d'agent d'imprégnation sur un non-tissé, un pansement ou un tissu.
